(19) **Europäisches Patentamt European Patent Office Office européen des brevets**

(11) **EP 4 589 021 A1**

(12) **EUROPEAN PATENT APPLICATION**

(43) Date of publication:
**23.07.2025 Bulletin 2025/30**

(21) Application number: **24382051.1**

(22) Date of filing: **19.01.2024**

(51) International Patent Classification (IPC):
***C12Q 1/6883*** $^{(2018.01)}$

(52) Cooperative Patent Classification (CPC):
**C12Q 1/6883;** C12Q 2600/158

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Barsande Salud S.L.
45005 Toledo (ES)**

(72) Inventor: **SÁNCHEZ-DEHESA RINCÓN, Marta
45005 Toledo (ES)**

(74) Representative: **Hoffmann Eitle
Hoffmann Eitle S.L.U.
Paseo de la Castellana 140, 3a planta
Edificio LIMA
28046 Madrid (ES)**

(54) **IN VITRO METHOD FOR THE DIAGNOSIS OF MALE INFERTILITY, OR FOR THE IDENTIFICATION OF THE FERTILITY STATUS OF A SUBJECT**

(57) The present invention refers to the fertility field. Particularly, the present invention refers to an *in vitro* method for the diagnosis of male infertility, or for the identification of the fertility status of a subject compared to a reference group, to an *in vitro* method for recommending a fertility treatment to a subject, and to an *in vitro* method for assessing DNA fragmentation in a biological sample obtained from a subject, which comprises assessing the expression of gamma-H2AX and H2AX in a sperm sample obtained from a subject, and processing the expression values to obtain a normalized expression value of gamma-H2AX with respect to the expression of H2AX.

**EP 4 589 021 A1**

**Description**

**FIELD OF THE INVENTION**

The present invention refers to the fertility field. Particularly, the present invention refers to an *in vitro* method for the diagnosis of male infertility, or for the identification of the fertility status of a subject compared to a reference group, to an *in vitro* method for recommending a fertility treatment to a subject, and to an *in vitro* method for assessing DNA fragmentation in a biological sample obtained from a subject, which comprises assessing the expression of gamma-H2AX and H2AX in a sperm sample obtained from a subject, and processing the expression values to obtain a normalized expression value of gamma-H2AX with respect to the expression of H2AX.

**STATE OF THE ART**

About 15% of couples of reproductive age have infertility problems, which are caused by male reproductive issues in 40% of the cases. However, throughout these years, the female factor has been the most studied and responsible, even without scientific evidence, for infertility. The male factor, on the other hand, is the forgotten factor in the equation, although this problem affects between 7-10% of males.

The known causes of male infertility are of diverse and multifactorial origin. The most frequent causes are considered to be varicocele, cryptorchidism, endocrine alterations (e. g. hypogonadism), immunological factors, obstructive physiological alterations, genetic alterations (cystic fibrosis, microdeletions of the Y chromosome), drug consumption, unhealthy lifestyle habits and idiopathic causes of unknown origin. The latter are estimated to account for 30%-40% of cases and represent a real problem in assisted reproduction treatments.

This high percentage is a consequence of the use of seminograms for the evaluation of male fertility, since these are unable to detect some of the causes of male infertility. Intracytoplasmic sperm injection (ICSI) was introduced in the clinical practice in 1992, to overcome male-factor infertility. After decades of routine use of this technique, the results are not as successful as expected. A possible explanation for this failure may be due to the fact that molecular alterations such as double-stranded sperm fragmentation or aneuploidy cannot be detected by seminograms and this series of alterations have been found to be present in 15% of patients classified as normozoospermic.

For this reason, in recent years the demand for extended studies for the evaluation of male infertility, beyond the semen analysis, has been increasing. In fact, the World Health Organization (WHO), in its sixth edition, includes the study of fragmentation, among others, as an extended diagnostic method for the male, considering it as a possible cause of male infertility.

There is controversy on the importance that sperm fragmentation may have at reproductive levels. There are those studies that relate a high fragmentation with a worse embryonic development, a lower implantation rate, a higher abortion rate and consequently a lower live birth rate. On the other hand, there are also studies that question the existence of this association.

The main problem that generates that the scientific community does not agree on this issue, is the variety of techniques, by which sperm fragmentation can be measured. There are methods capable of measuring the total sperm fragmentation index, encompassing double-chain and single-chain breaks, such as TUNEL, SCSA or Dispersion, more accurate because they use flow cytometry and analyze a large number of spermatozoa, but less specific with the diagnosis of double-chain fragmentation. On the other hand, tests that are able to discern between both breaks, such as neutral comet and alkaline comet, but very limited by the small number of spermatozoa studied.

This variety of techniques, each with a detection threshold, an intra-laboratory sensitivity/specificity, and a dubious correlation between them, means that to date, there is no reliable standard test for the correct detection of sperm double-strand fragmentation.

So, there is an unmet medical need of finding strategies aimed at the identification of sperm DNA fragmentation, male infertility or reduced fertility compared to a reference group in a subject. The present invention is focused on solving this problem, and *in vitro* methods for the diagnosis of male infertility, for the identification of the fertility status of a subject compared to a reference group, and for assessing DNA fragmentation in a biological sample are herein provided.

**DESCRIPTION OF THE INVENTION**

**Brief description of the invention**

As explained above, the present invention refers to an *in vitro* method for the diagnosis of male infertility, or for the identification of the fertility status of a subject compared to a reference group, to an *in vitro* method for recommending a fertility treatment to a subject, and to an *in vitro* method for assessing DNA fragmentation in a biological sample obtained from a subject, which comprises assessing the expression of gamma-H2AX and H2AX in a sperm sample obtained from a subject, and processing the expression values to obtain a normalized expression value of gamma-H2AX with respect to the expression of H2AX.

The inventors of the present invention have developed a method for the identification of male infertility based on the detection of DNA fragmentation, since DNA fragmentation in sperm has been described to be associated to male infertility. This method is based on the assessment of gamma-H2AX expression, a well-known biomarker of DNA double-strand breaks (DSB).

The inventors identified a major limitation associated to the use of this biomarker for the detection of male infertility that had been overlooked by the scientific community, which is the fact that immature sperm cells have a higher abundance of H2AX, meaning that they can present a higher abundance of gamma-H2AX even in the absence of high DBS damage. Thus, quantifying the abundance of gamma-H2AX in absolute values could incorrectly indicate the presence of DNA damage in immature sperm cells. As a consequence, subjects may be incorrectly diagnosed with male infertility. The inventors of the present invention realized that this problem could be solved by normalizing the expression of gamma-H2AX with respect to the total H2AX. This in turn provides a higher resolution of the DSB damage present in immature sperm cells, and consequently a more precise diagnosis of male infertility.

The present invention provides a number of methods based on the use of this normalization step that could be used for the assessment of DNA fragmentation **(Example 2.1),** for the diagnosis of male infertility or for the identification of the fertility status of a subject compared to a reference group **(Example 2.2),** or for recommending a fertility treatment in a subject **(Example 2.3).** All these methods represent improved strategy compared to the use of absolute gamma-H2AX expression values.

The first embodiment of the invention refers to an *in vitro* method for the diagnosis of male infertility, or for the identification of the fertility status of a subject compared to a reference group, which comprises:

a) assessing the expression of gamma-H2AX and H2AX in a sperm sample obtained from a subject,
b) processing the expression values to obtain a normalized expression value of gamma-H2AX with respect to the expression of H2AX,
c) wherein an increased normalized expression value of gamma-H2AX with respect to the expression of H2AX, as compared to a pre-established threshold value measured in sperm samples from fertile subjects, is indicative that the subject is infertile,
d) wherein an increased normalized expression value of gamma-H2AX with respect to the expression of H2AX, as compared to a pre-established threshold value measured in sperm samples from a reference group, is indicative that the subject has a decreased fertility compared to the reference group,
e) wherein an increased coefficient of variation of the normalized expression value of gamma-H2AX with respect to the expression of H2AX, as compared to a pre-established threshold value measured in sperm samples from fertile subjects, is indicative that the subject is infertile, or
f) wherein an increased coefficient of variation of the normalized expression value of gamma-H2AX with respect to the expression of H2AX, as compared to a pre-established threshold value measured in sperm samples from a reference group, is indicative that the subject has a decreased fertility compared to the reference group.

In a preferred embodiment, the *in vitro* method of the first embodiment further comprises:

i) determining the percentage of sub-haploid cells in a sperm sample obtained from a subj ect,
ii) wherein an increased percentage of sub-haploid cells, as compared to a pre-established threshold value measured in sperm samples from fertile subjects, is indicative that the subject is infertile, or
iii) wherein an increased percentage of sub-haploid cells, as compared to a pre-established threshold value measured in sperm samples from a reference group, is indicative that the subject has a decreased fertility compared to the reference group.

The second embodiment of the invention refers to an *in vitro* method for recommending a fertility treatment to a subject, which comprises:

a) assessing the expression of gamma-H2AX and H2AX in a sperm sample obtained from a subject,
b) processing the expression values to obtain a normalized expression value of gamma-H2AX with respect to the expression of H2AX,
c) wherein an increased normalized expression value of gamma-H2AX with respect to the expression of H2AX, as compared to a pre-established threshold value measured in sperm samples from fertile subjects, is indicative that a fertility treatment is recommended, or
a) wherein an increased coefficient of variation of the normalized expression value of gamma-H2AX with respect to the expression of H2AX, as compared to a pre-established threshold value measured in sperm samples from fertile subjects, is indicative that a fertility treatment is recommended.

In a preferred embodiment, the *in vitro* method of the second embodiment further comprises:

i) determining the percentage of sub-haploid cells in a sperm sample obtained from a subj ect,
ii) wherein an increased percentage of sub-haploid cells, as compared to a pre-established threshold value measured in sperm samples from fertile subjects, is indicative that a fertility treatment is recommended.

The third embodiment of the invention refers to an *in vitro* method for assessing DNA fragmentation in a biological sample obtained from a subject, which comprises:

a) assessing the expression of gamma-H2AX and H2AX in a biological sample obtained from a subject,
b) processing the expression values to obtain a normalized expression value of gamma-H2AX with respect to the expression of H2AX,
c) wherein the normalized expression value of gamma-H2AX with respect to the expression of H2AX is indicative of the amount of DNA fragmentation of the biological sample,
d) wherein an increased normalized expression value of gamma-H2AX with respect to the expression of H2AX, as compared to a pre-established threshold value measured in samples from a reference group, is indicative that the sample has an increased DNA fragmentation compared to the reference group,
e) wherein the coefficient of variation of the normalized expression value of gamma-H2AX with respect to the expression of H2AX is indicative of the amount of DNA fragmentation of the biological sample, or
f) wherein an increased coefficient of variation of the normalized expression value of gamma-H2AX with respect to the expression of H2AX, as compared to a pre-established threshold value measured in samples from a reference group, is indicative that the sample has an increased DNA fragmentation compared to the reference group.

In a preferred embodiment, the *in vitro* method of the third embodiment further comprises:

i) determining the percentage of sub-haploid cells in a sperm sample obtained from a subj ect,
ii) wherein the percentage of sub-haploid cells is indicative of the amount of DNA fragmentation of the biological sample,
iii) wherein an increased percentage of sub-haploid cells, as compared to a pre-established threshold value measured in samples from a reference group, is indicative that the sample has an increased DNA fragmentation compared to the reference group.

In a preferred embodiment, the biological sample is sperm.
In a preferred embodiment, the normalized expression value of gamma-H2AX with respect to the expression of H2AX is characterized in that it is measured in a population of cells wherein at least 70%, 72%, 74%, 76%, 78%, 80%, 82%, 84%, 86%, 88%, 90%, 92%, 94%, 96%, 97%, 98% or 99% of the cells are haploid.
In a preferred embodiment, steps a) and/or i) are performed by flow cytometry.

In a preferred embodiment, step b) is performed using the following formula: $\dfrac{MFI\ of\ \gamma H2AX\ in\ \gamma H2AX^{+}\ cells}{MFI\ of\ H2AX\ in\ H2AX^{+}\ cells}$, wherein MFI stands for mean fluorescence intensity.
The fourth embodiment of the invention refers to a fertility treatment for use in the treatment of infertility, to promote fertility or to increase the probabilities of conceiving a child, wherein the method comprises assessing whether a subject suffers from male infertility or whether a fertility treatment is recommended in a subject by following the method of the second embodiment.
In a preferred embodiment, the fertility treatment is selected from: *in vitro* fertilization, intracytoplasmic sperm injection (ICSI), preimplantation genetic testing (PGT), preferably preimplantation genetic testing for aneuploidy (PGT-A), microfluidics, annexin columns, oral treatment with curcuma and piperine and gamete change.
In a preferred embodiment the fertility treatment is for use in the treatment of male infertility.
In a preferred embodiment the fertility treatment is for use to promote fertility in a male.
The fifth embodiment of the invention refers to the *in vitro* use of the proteins H2AX and gamma-H2AX, or of a kit comprising reagents for the assessment of the expression of the combination of the proteins H2AX and gamma-H2AX for the diagnosis of male infertility, or for the identification of the fertility status of a subject compared to a reference group, for recommending a fertility treatment to a subject, or for assessing DNA fragmentation in a biological sample obtained from a subject, preferably wherein the biological sample is semen.
In a preferred embodiment, the proteins are used in combination with the normalized expression value of gamma-H2AX with respect to the expression of H2AX, and/or with the coefficient of variation of the normalized expression value of gamma-H2AX with respect to the expression of H2AX.

In a preferred embodiment, the kit also comprises reagents for the assessment of the normalized expression value of gamma-H2AX with respect to the expression of H2AX, and/or with the coefficient of variation of the normalized expression value of gamma-H2AX with respect to the expression of H2AX.

In a preferred embodiment, the proteins are used in combination with the percentage of sub-haploid cells, preferably the percentage of sub-haploid cells in a sperm sample.

In a preferred embodiment, the kit also comprises reagents for the assessment of the percentage of sub-haploid cells, preferably the percentage of sub-haploid cells in a sperm sample.

In a preferred embodiment the subject is a male subject.

In a preferred embodiment, the coefficient of variation is the robust coefficient of variation.

The present invention also refers to a computer-implemented invention, wherein a processing unit (hardware) and a software are configured to:

i) receive the normalized expression value of gamma-H2AX with respect to the expression of H2AX, the coefficient of variation of the normalized expression value of gamma-H2AX with respect to the expression of H2AX, and/or the percentage of sub-haploid cells,
ii) process these values in search of substantial variations or deviations with respect to a reference group, and
iii) provide an output through a terminal display of the variation or deviation of the level of expression values with respect to the reference group, wherein the variation or deviation of the one or more values is indicative that the subject is infertile, that the subject has a decreased fertility compared to the reference group, that a fertility treatment is recommended, of the amount of DNA fragmentation of the biological sample, and/or that the sample has an increased DNA fragmentation compared to the reference group.

The present invention also refers to a computer program or a computer-readable media containing means for carrying out a method as herein defined.

In the context of the present invention the following terms are defined:

• The term "comprising" means including, but it is not limited to, whatever follows the word "comprising". Thus, use of the term "comprising" indicates that the listed elements are required or mandatory, but that other elements are optional and may or may not be present.
• The term "consisting of" means including, and it is limited to, whatever follows the phrase "consisting of". Thus, the phrase "consisting of' indicates that the listed elements are required or mandatory, and that no other elements may be present.
• The term "male infertility" refers to a disease of the male reproductive system that lowers the chances to conceive a child. It means you cannot start a pregnancy with your female partner. Male infertility can be caused by low sperm production, abnormal sperm function or blockages that prevent the delivery of sperm. Illnesses, injuries, chronic health problems, lifestyle choices and other factors may contribute to male infertility.
• The term "fertility status of a subject" refers to the ability to conceive a child. If a subject presents a decreased fertility status compared to a reference group, it means their ability to conceive a child is lower that of the reference group on average. In this case, since the fertility status is assessed using a sperm sample, the subject is a male subject, or a sperm-bearing subject.
• The term "normalized expression value of gamma-H2AX with respect to the expression of H2AX" preferably involves using a ratio or a percentage.
• The term "fertility treatment" in the context of the present invention refers to any compound, composition or *in vitro* strategy that could increase the chances of a male subject to conceive a child. Thus, it does not only include compounds that may be used to increase sperm quality, but also *in vitro* methods that could increase his chances to conceive a child without necessarily improving sperm quality, such as *in vitro* fertilization, intracytoplasmic sperm injection (ICSI), or preimplantation genetic testing (PGT), such as PGT for aneuploidy, which is used to test an embryo for genetic diseases before the embryo is transferred.

## Detailed description of the invention

The present invention is illustrated by means of the Examples set below without the intention of limiting its scope of protection.

## Example 1. Material and Methods

**Example 1.1. Selection of subjects for the study**

The study was performed using semen samples from 35 healthy, fertile male sperm donors withdrawn from the CEIFER Biobank (with number of legal live births obtained n=6) (control sample). Patient samples were obtained from the "Assisted Reproduction Unit of HM IMI Toledo Fertility Center".

The inclusion criteria were as follows: presence of $\geq$ 4 x 106 total spermatozoa in the sample, normal karyotype (46XY) and signature of the informed consent.

Exclusion criteria for all patients included: refusal to sign the informed consent, altered karyotype, oligozoospermia with a total sperm count less than 4 x 106, and leucospermia.

The study was performed in accordance with the Declaration of Helsinki and with the favourable opinion of the Ethics Committee of the Hospital Nuestra Señora del Prado de Talavera de la Reina (Toledo) with code 3/2020. In addition, data collection was carried out in accordance with the Organic Law on Personal Data Protection and Guarantee of Digital Rights. In all cases, an informed consent was obtained from the patients before their inclusion in the study.

**Example 1.2. Sample collection and experimental design**

Fresh semen samples from patients of the "Assisted Reproduction Unit of HM IMI Toledo Fertility Center (n=)" were obtained by masturbation, after 48-72h of sexual abstinence. After total seminal liquefaction, the samples were evaluated by conventional seminogram, according to the indications of the latest WHO manual (6th edition).

Both study groups were analyzed by flow cytometry, using the H2AX fragmentation study protocol. It was assumed that healthy donors had normal double-chain fragmentation, given their reproductive quality.

**Example 1.3. Sample preparation and analysis by flow cytometry**

For the study of total H2AX and VH2AX, a protocol for fixation and marking of samples was developed by the laboratory in collaboration with the Flow Cytometry Research Support Service of the Hospital Nacional de Parapléjicos.

The materials used for this step were:

- PBS-BSA 0.5 % $\rightarrow$ PBS-BSA

- Paraformaldehyde 3.8%

- PBS-BSA 0,5 %-Triton 0.1% $\rightarrow$ PBS-BSA-Triton

- EtOH 70%

- Antibody PE anti-H2A.X Phospho (Ser139) Clone 2F3 (Biolegend) $\rightarrow$ anti-yH2AX-PE

- Antibody Alexa Fluor® anti-H2A.X Clone W16171A (Biolegend) $\rightarrow$ anti-H2AX-A647

- Propidium iodide/RNase (InmunoStep) $\rightarrow$ IP/RNase

- Draq 5 (Biolegend)

The inventors used the following protocol:

1. Thaw the sperm cells (spz) at room temperature in a 15 mL falcon.
2. Wash with 4mL of PBS-BSA.
3. Centrifuge cells, to remove cryoprotectant at 300g for 5 minutes (min). Resuspend in PBS-BSA to a concentration of 1x106cells/100ul (per test sample, set up 3 tubes).

   a. Negative control
   b. Sample.1
   c. Sample.2

4. Fixation. We add 100ul PFA 3.6 % to each tube (final PFA 1.8%).
5. Vortex 1-2 seconds (s) and incubate 15min at room temperature (RT).
6. Vortex again 1-2 s and add PBS-BSA

7. Centrifuge at 300g for 5 min.

8. Permeabilize in 70% EtOH(E): remove supernatant and resuspend in residual fluid. Add 700ul of EtOH cooled to -20°C, dropwise with Vortex at low speed. Incubate at - 20°C overnight.

9. The next morning stop with 2ml of PBS-BSA and centrifuge at 300g 5min removing supernatant with aspiration. Leave some liquid and transfer to 1.5 mL tubes. Repeat centrifugation. This second washing, with 1 ml of PBS-BSA-Triton

10. Prepare antibody mix for staining (n samples+2) in PBS-BSA-Triton.

   a. H2AX: final concentration 6.25 ng/ml
   b. Total H2AX: final concentration 2.5 $\mu$g/ml

11. Staining. Immediately add 100 $\mu$l of antibody mix to each tube.

12. Vortex (1-2 s) and incubate 1 hour at RT protected from light.

13. Wash. Add 1 mL of PBS-BSA-Triton. Centrifuge the cells at 300g for 5min at RT and remove the supernatant by aspiration. Repeat.

14. Remove supernatant and resuspend each sample in DNA staining solution.

VH2AX (phosphorylated at Ser139) staining:

15. Prepare DRAQ5 mix: prepare 1:750 dilution in PBS-BSA.

16. Resuspend the spz pellet in 150 $\mu$l of Draq 5.

17. Incubate 15 min at RT in the dark.

18. Analyze on the CytoFLEX S cytometer by tube acquisition.

H2AX staining

15. Resuspend the spz pellet in 250 $\mu$l of IP/RNase.

16. Incubate 15 min at RT in the dark.

17. Analyze on the CytoFLEX S cytometer by tube acquisition.

Sample acquisition using a CytoFLEX S cytometer

After starting up the cytometer, following the daily switch-on protocols, we proceeded to perform the quality control of the equipment status following the manufacturer's instructions, using the CytoFLEX Daily QC Fluorospheres (Beckman Coulter).

To reduce the batch effect that can occur when performing measurements on different days, the gains of the detectors were adjusted daily using 8-peak Rainbow fluorospheres (BD Biosciences).

The detectors used for the detection of the different reagents were:

- FSC: forward scatter. Linear scale
- SSC (BP 488/8): orthogonal scattering. Biexponential scale
- PE (BP 585/42): γH2AX / IP. Biexponential scale
- PerCP (BP 690/50): Draq 5. biexponential scale.
- APC (BP 660/10): H2AX total. Biexponential scale

Samples were acquired at a constant rate of 60 $\mu$l/min (fast) and with a fixed volume: 100 $\mu$l for γH2AX and 150 $\mu$l for total H2AX (60-66 % of the total sample volume).

Analysis

First, cell aggregates were removed by comparing the area (A) with the height (H) of the FSC pulse, drawing the singlets region. Next, the forward scatter (FSC) and orthogonal scatter (SSC) in the events of the "singlets" region were analyzed to analyze the size and complexity of the sample. The region belonging to the spz is established by removing the cellular debris present in the samples. To eliminate from the analysis doublets and aggregates of cells that might have escaped the first selection and could be interpreted as diploid cells, the area (A) and the width (W) of the DNA staining signal (IP on the PE detector, for the analysis of total H2AX, and Draq 5 on the PerCP detector, for the analysis of $\gamma$H2AX) were confronted. Next, the DNA content in the events contained in the previously selected region of nuclei without doublets was analyzed using a histogram. The following subpopulation classification was performed:

- Sub-haploid population (<n): spz with lower DNA content than expected in healthy spz (23 chromosomes). They correspond to apoptotic spz that have started to fragment their DNA.
- Haploid population (n): spz with normal DNA content.
- Haploid/diploid + population (+n/2n): spz with increased DNA content, i.e. with additional chromosomes (more than 23).

Analysis for the presence of total H2AX and $\gamma$H2AX (phosphorylated form indicative of double-stranded fragmentation, DSB) is performed on the haploid population (n).

To establish the positive population for both total H2AX and $\gamma$H2AX, a negative control of H2AXtot/$\gamma$H2AX staining was used, which is the same sample without the addition of the antibodies.

All samples were measured in duplicate.

The data to be analyzed are as follows:

- Percentage of total H2AX: percentage of haploid spz that have H2AX in their DNA. The higher the percentage, the more immature they are, since a higher histone content indicates a lower turnover by protamines.
- Percentage of $\gamma$H2AX: percentage of haploid spz that have phosphorylated H2AX indicative of DSB. The higher the percentage, the higher the number of damaged spz. There is a certain level of normal basal H2AX phosphorylation in all cells. Therefore, this must be combined with the measurement of fluorescence intensity (see below).
- Median Fluorescence Intensity (MFI) of total H2AX: The fluorescence intensity changes as a function of the amount of histone present in the spz. A higher fluorescence intensity is indicative of a higher number of histones present in the spz and, therefore, a lower turnover by protamines, indicating immaturity of the spz.
- Median Fluorescence Intensity (MFI) of $\gamma$H2AX: The fluorescence intensity changes as a function of the number of cleavage sites. The higher the fragmentation, the higher the fluorescence intensity of $\gamma$H2AX.
- Percentage of +2n region, to establish the level of sperm ploidy. Given that the majority of sperm cells of a healthy male should occupy the n region (haploid), a cutoff point can be established to determine which males have a higher-than-expected percentage in the diploid region (2n) and in the +n region (more than haploid).
  This diagnosis will allow us to refer patients to embryonic genetic selection techniques (PGT-A), since the presence of spermatozoa with altered ploidy is related to a greater number of embryonic chromosomal alterations.

Another measurable parameter in the haploid region is the robust coefficient of variation (rCV) of the haploid region, which helps to establish the homogeneity of the sample in terms of sperm DNA structure. In other words, more fragmented samples will present a more dispersed distribution of the haploid region (higher CV) than less damaged samples.

In addition, the analysis of the percentage of spz in the sub-haploid region gives an idea of the extensive fragmentation of the sample, both single-stranded and DSB, being higher in samples with high damage.

To correlate the actual damage of a sample and provide a diagnosis to the patient, the data from the $\gamma$H2AX measurements are related to the data from the total H2AX measurement. In this way, the initial starting compaction and maturity state and points susceptible to damage will be seen. To normalize the damage measured by increasing $\gamma$H2AX with respect to the total H2AX content, the following ratio is established:

$$\frac{\gamma H2AX}{H2AX} = \frac{\text{MFI of } \gamma H2AX \text{ of haploid } \gamma H2AX^+ \text{spz}}{\text{MFI of H2AX of haploid H2AX}^+ \text{ spz}}$$

Once we have the data, on $\gamma$H2AX (damage signal) we will be able to make the final fragmentation ratio, versus the initial predisposition. The higher the damage, the higher the MFI $\gamma$H2AX and the ratio will increase.

This normalization will mean that those spz that are more immature and contain more total H2AX, but do not have as much DSB damage, will not be identified as damaged because they have more phosphorylated H2AX.

**Example 2. Applications**

**Example 2.1. Assessing DNA fragmentation in a biological sample obtained from a subject**

It is known that DNA double-strand breaks (DSB) induce H2Ax phosphorylation, and gamma-H2AX has been described as a biomarker for DNA DSBs. Thus, it is plausible that the procedure described in the materials and methods section may be used for the assessment of DNA fragmentation in a biological sample.

The procedure described in the present invention can be implemented to assess of DNA fragmentation in the following manner:

The assessment of DNA fragmentation in a biological sample obtained from a subject can be performed by assessing the expression of gamma-H2AX and H2AX in a biological sample obtained from a subject and processing the expression values to obtain a normalized expression value of gamma-H2AX with respect to the expression of H2AX. An increased normalized expression value of gamma-H2AX with respect to the expression of H2AX will be indicative of the amount of DNA fragmentation of the biological sample. Similarly, an increased normalized expression value of gamma-H2AX with respect to the expression of H2AX, as compared to a pre-established threshold value measured in samples from a reference group, will be indicative that the sample has an increased DNA fragmentation compared to the reference group.

The normalized expression value of gamma-H2AX with respect to the expression of H2AX may be calculated using the following formula:

$$\frac{\gamma H2AX}{H2AX} = \frac{\text{MFI of } \gamma H2AX \text{ of haploid } \gamma H2AX^{+}\text{spz}}{\text{MFI of H2AX of haploid H2AX}^{+} \text{ spz}}$$

The higher the damage, the higher the MFI $\gamma$H2AX, and consequently the higher ratio.

The person skilled in the art would be able to develop other alternative methods to calculate the normalized expression value of gamma-H2AX with respect to the expression of H2AX.

The assessment of DNA fragmentation in a biological sample obtained from a subject can be performed by assessing the coefficient of variation of the normalized expression value of gamma-H2AX with respect to the expression of H2AX. This value can be used to establish the homogeneity of the sample in terms of sperm DNA structure. In other words, more fragmented samples will present a more dispersed distribution of the haploid region (higher CV) than less damaged samples. Thus, an increased coefficient of variation of the normalized expression value of gamma-H2AX with respect to the expression of H2AX will be indicative of the amount of DNA fragmentation of the biological sample. Similarly, an increased coefficient of variation of the normalized expression value of gamma-H2AX with respect to the expression of H2AX, as compared to a pre-established threshold value measured in samples from a reference group, will be indicative that the sample has an increased DNA fragmentation compared to the reference group.

The assessment of the normalized expression value of gamma-H2AX with respect to the expression of H2AX or of the coefficient of variation of the normalized expression value of gamma-H2AX with respect to the expression of H2AX may be preferably performed in the haploid population (n).

One of the most important elements of the procedure herein proposed is the normalization step. Immature sperm cells have a higher abundance of H2AX. Therefore, they can present a higher amount of gamma-H2AX even in the absence of high DSB damage. Normalizing the expression of gamma-H2AX with respect to the total H2AX provides a higher resolution of the DSB damage present in immature sperm cells.

The percentage of sub-haploid cells in a sperm sample obtained from a subject is also indicative of the extensive fragmentation of the sample, both single-stranded and DSB, being higher in samples with high damage. Thus, the percentage of sub-haploid cells is indicative of the amount of DNA fragmentation of the biological sample. Similarly, an increased percentage of sub-haploid cells, as compared to a pre-established threshold value measured in samples from a reference group, is indicative that the sample has an increased DNA fragmentation compared to the reference group.

**Example 2.2. Diagnosis of male infertility and identification of the fertility status of a subject compared to a reference group**

DNA fragmentation has been widely associated to male infertility, or to the presence of a decreased fertility status. Thus, it is plausible that the procedures described in **Example 2.1** may be used for the diagnosis of male infertility, as well as for the identification of the fertility status of a subject compared to a reference group.

These procedures can be implemented to diagnose infertility and to identify the fertility status of a subject in the following manner:

The diagnosis of male infertility can be achieved by assessing the expression of gamma-H2AX and H2AX in a sperm sample obtained from a subject and processing the expression values to obtain a normalized expression value of gamma-H2AX with respect to the expression of H2AX. An increased normalized expression value of gamma-H2AX with respect to the expression of H2AX, as compared to a pre-established threshold value measured in sperm samples from fertile subjects, will be indicative that the subject is infertile. Similarly, an increased normalized expression value of gamma-H2AX with respect to the expression of H2AX, as compared to a pre-established threshold value measured in sperm samples from a reference group, will be indicative that the subject has a decreased fertility compared to the reference group.

The normalized expression value of gamma-H2AX with respect to the expression of H2AX may be calculated using the following formula:

$$\frac{\gamma H2AX}{H2AX} = \frac{\text{MFI of } \gamma H2AX \text{ of haploid } \gamma H2AX^+ \text{spz}}{\text{MFI of H2AX of haploid H2AX}^+ \text{ spz}}$$

The higher the damage, the higher the MFI $\gamma$H2AX, and consequently the higher ratio.

The person skilled in the art would be able to develop other alternative methods to calculate the normalized expression value of gamma-H2AX with respect to the expression of H2AX.

The coefficient of variation of the normalized expression value of gamma-H2AX with respect to the expression of H2AX may also be used for this purpose. An increased coefficient of variation of the normalized expression value of gamma-H2AX with respect to the expression of H2AX, as compared to a pre-established threshold value measured in sperm samples from fertile subjects, will be indicative that the subject is infertile. Similarly, an increased coefficient of variation of the normalized expression value of gamma-H2AX with respect to the expression of H2AX, as compared to a pre-established threshold value measured in sperm samples from a reference group, will be indicative that the subject has a decreased fertility compared to the reference group.

As explained for the assessment of DNA fragmentation **(Example 2.1),** the assessment of the normalized expression value of gamma-H2AX with respect to the expression of H2AX or of the coefficient of variation of the normalized expression value of gamma-H2AX with respect to the expression of H2AX may be preferably performed in the haploid population (n).

An increased percentage of sub-haploid cells, as compared to a pre-established threshold value measured in sperm samples from fertile subjects, will also be indicative that the subject is infertile. Similarly, an increased percentage of sub-haploid cells, as compared to a pre-established threshold value measured in sperm samples from a reference group, will be indicative that the subject has a decreased fertility compared to the reference group.

### Example 2.3. Recommending a fertility treatment to a subject

It is important to note that there are a wide variety of fertility treatments. Some of them are directed towards improving sperm quality, which may be desirable in males that suffer from male infertility or in males in which the fertility status is below an optimal range (i.e. subjects that have a decreased fertility compared to a reference group). Other treatments are not directed towards improving sperm quality, but rather towards selecting the best performing sperm cells, or testing embryos for specific diseases prior to implanting them into the uterus of the mother. These strategies may be desirable for couples wherein the male has been diagnosed with low fertility or a poor DNA quality since they could pose a higher chance of passing diseases to their offspring.

Since the procedures described in **Example 2.2** can be used for the diagnosis of male infertility, as well as for the identification of the fertility status of a subject compared to a reference group, they can also be used for recommending a fertility treatment.

### Claims

1. *In vitro* method for the diagnosis of male infertility, or for the identification of the fertility status of a subject compared to a reference group, which comprises:

   a) assessing the expression of gamma-H2AX and H2AX in a sperm sample obtained from a subject,
   b) processing the expression values to obtain a normalized expression value of gamma-H2AX with respect to the expression of H2AX,

c) wherein an increased normalized expression value of gamma-H2AX with respect to the expression of H2AX, as compared to a pre-established threshold value measured in sperm samples from fertile subjects, is indicative that the subject is infertile,

d) wherein an increased normalized expression value of gamma-H2AX with respect to the expression of H2AX, as compared to a pre-established threshold value measured in sperm samples from a reference group, is indicative that the subject has a decreased fertility compared to the reference group,

e) wherein an increased coefficient of variation of the normalized expression value of gamma-H2AX with respect to the expression of H2AX, as compared to a pre-established threshold value measured in sperm samples from fertile subjects, is indicative that the subject is infertile, or

f) wherein an increased coefficient of variation of the normalized expression value of gamma-H2AX with respect to the expression of H2AX, as compared to a pre-established threshold value measured in sperm samples from a reference group, is indicative that the subject has a decreased fertility compared to the reference group.

2. *In vitro* method, according to claim 1, which further comprises:

i) determining the percentage of sub-haploid cells in a sperm sample obtained from a subj ect,

ii) wherein an increased percentage of sub-haploid cells, as compared to a pre-established threshold value measured in sperm samples from fertile subjects, is indicative that the subject is infertile, or

iii) wherein an increased percentage of sub-haploid cells, as compared to a pre-established threshold value measured in sperm samples from a reference group, is indicative that the subject has a decreased fertility compared to the reference group.

3. *In vitro* method for recommending a fertility treatment to a subject, which comprises:

a) assessing the expression of gamma-H2AX and H2AX in a sperm sample obtained from a subject,

b) processing the expression values to obtain a normalized expression value of gamma-H2AX with respect to the expression of H2AX,

c) wherein an increased normalized expression value of gamma-H2AX with respect to the expression of H2AX, as compared to a pre-established threshold value measured in sperm samples from fertile subjects, is indicative that a fertility treatment is recommended, or

b) wherein an increased coefficient of variation of the normalized expression value of gamma-H2AX with respect to the expression of H2AX, as compared to a pre-established threshold value measured in sperm samples from fertile subjects, is indicative that a fertility treatment is recommended.

4. *In vitro* method, according to claim 3, which further comprises:

i) determining the percentage of sub-haploid cells in a sperm sample obtained from a subj ect,

ii) wherein an increased percentage of sub-haploid cells, as compared to a pre-established threshold value measured in sperm samples from fertile subjects, is indicative that a fertility treatment is recommended.

5. *In vitro* method for assessing DNA fragmentation in a biological sample obtained from a subject, which comprises:

a) assessing the expression of gamma-H2AX and H2AX in a biological sample obtained from a subject,

b) processing the expression values to obtain a normalized expression value of gamma-H2AX with respect to the expression of H2AX,

c) wherein the normalized expression value of gamma-H2AX with respect to the expression of H2AX is indicative of the amount of DNA fragmentation of the biological sample,

d) wherein an increased normalized expression value of gamma-H2AX with respect to the expression of H2AX, as compared to a pre-established threshold value measured in samples from a reference group, is indicative that the sample has an increased DNA fragmentation compared to the reference group,

e) wherein the coefficient of variation of the normalized expression value of gamma-H2AX with respect to the expression of H2AX is indicative of the amount of DNA fragmentation of the biological sample, or

f) wherein an increased coefficient of variation of the normalized expression value of gamma-H2AX with respect to the expression of H2AX, as compared to a pre-established threshold value measured in samples from a reference group, is indicative that the sample has an increased DNA fragmentation compared to the reference group.

6. *In vitro* method, according to claim 5, which further comprises:

   i) determining the percentage of sub-haploid cells in a sperm sample obtained from a subj ect,
   ii) wherein the percentage of sub-haploid cells is indicative of the amount of DNA fragmentation of the biological sample,
   iii) wherein an increased percentage of sub-haploid cells, as compared to a pre-established threshold value measured in samples from a reference group, is indicative that the sample has an increased DNA fragmentation compared to the reference group.

7. *In vitro* method, according to any of the claims 5 or 6, wherein the biological sample is sperm.

8. *In vitro* method, according to any of the previous claims, wherein the normalized expression value of gamma-H2AX with respect to the expression of H2AX is **characterized in that** it is measured in a population of cells wherein at least 70%, 72%, 74%, 76%, 78%, 80%, 82%, 84%, 86%, 88%, 90%, 92%, 94%, 96%, 97%, 98% or 99% of the cells are haploid.

9. *In vitro* method, according to any of the previous claims, wherein steps a) and/or i) are performed by flow cytometry.

10. *In vitro* method, according to any of the previous claims, wherein the step b) is performed using the following formula:

$$\frac{MFI\ of\ \gamma H2AX\ in\ \gamma H2AX^{+}\ cells}{MFI\ of\ H2AX\ in\ H2AX^{+}\ cells}$$, wherein MFI stands for mean fluorescence intensity.

11. Fertility treatment for use in the treatment of infertility, to promote fertility or to increase the probabilities of conceiving a child, wherein the method comprises assessing whether a subject suffers from male infertility or whether a fertility treatment is recommended in a subject by following the method of claims 3 or 4.

12. Fertility treatment, according to claim 11, selected from: *in vitro* fertilization, intracytoplasmic sperm injection (ICSI), preimplantation genetic testing (PGT), preferably preimplantation genetic testing for aneuploidy (PGT-A), microfluidics, annexin columns, oral treatment with curcuma and piperine and gamete change.

13. *In vitro* use of the proteins H2AX and gamma-H2AX, or of a kit comprising reagents for the assessment of the expression of the combination of the proteins H2AX and gamma-H2AX for the diagnosis of male infertility, or for the identification of the fertility status of a subject compared to a reference group, for recommending a fertility treatment to a subject, or for assessing DNA fragmentation in a biological sample obtained from a subject, preferably wherein the biological sample is semen.

14. *In vitro* use or *in vitro* use of a kit, according to claim 13, wherein the proteins are used in combination with the normalized expression value of gamma-H2AX with respect to the expression of H2AX, and/or with the coefficient of variation of the normalized expression value of gamma-H2AX with respect to the expression of H2AX, or wherein the kit also comprises reagents for the assessment of the normalized expression value of gamma-H2AX with respect to the expression of H2AX, and/or with the coefficient of variation of the normalized expression value of gamma-H2AX with respect to the expression of H2AX.

15. *In vitro* use or *in vitro* use of a kit, according to any of the claims 13 or 14, wherein the proteins are used in combination with the percentage of sub-haploid cells, preferably the percentage of sub-haploid cells in a sperm sample, or wherein the kit also comprises reagents for the assessment of the percentage of sub-haploid cells, preferably the percentage of sub-haploid cells in a sperm sample.

**Europäisches Patentamt**
**European Patent Office**
**Office européen des brevets**

# EUROPEAN SEARCH REPORT

Application Number

EP 24 38 2051

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LLAVANERA MARC ET AL: "A systematic review identifying fertility biomarkers in semen: a clinical approach through Omics to diagnose male infertility", FERTILITY AND STERILITY, ELSEVIER, AMSTERDAM, NL, vol. 118, no. 2, 17 June 2022 (2022-06-17) , pages 291-313, XP087126882, ISSN: 0015-0282, DOI: 10.1016/J.FERTNSTERT.2022.04.028 [retrieved on 2022-06-17] p. 298; * abstract; table 1 * | 1-15 | INV. C12Q1/6883 |
| A | ZHONG HUI-ZHI ET AL: "Evaluating [gamma]H2AX in spermatozoa from male infertility pati", FERTILITY AND STERILITY, ELSEVIER, AMSTERDAM, NL, vol. 104, no. 3, 7 July 2015 (2015-07-07), pages 574-581, XP029262671, ISSN: 0015-0282, DOI: 10.1016/J.FERTNSTERT.2015.06.004 * the whole document * * figures 1D, 2D; table 1 * | 1-15 | |
| | | | **TECHNICAL FIELDS SEARCHED (IPC)** |
| | | | C12Q |
| A | GAROLLA ANDREA ET AL: "FSH treatment in infertile males candidate to assisted reproduction improved sperm DNA fragmentation and pregnancy rate", ENDOCRINE, HUMANA PRESS, INC, US, vol. 56, no. 2, 27 July 2016 (2016-07-27), pages 416-425, XP036214263, ISSN: 1355-008X, DOI: 10.1007/S12020-016-1037-Z [retrieved on 2016-07-27] * abstract; figure 1 * | 1-15 | |

The present search report has been drawn up for all claims

| Place of search | Date of completion of the search | Examiner |
|---|---|---|
| Munich | 24 June 2024 | Costa Roldán, Nuria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)